Europäisches Patentamt

European Patent Office

Office européen des brevets

(11) Veröffentlichungsnummer: **0 315 795 B1**

# EUROPÄISCHE PATENTSCHRIFT

(45) Veröffentlichungstag der Patentschrift: **23.10.91**

(21) Anmeldenummer: **88117210.0**

(22) Anmeldetag: **15.10.88**

(51) Int. Cl.⁵: **A61F 2/34**

(54) **Hüftgelenkspfanne.**

(30) Priorität: **11.11.87 CH 4398/87**

(43) Veröffentlichungstag der Anmeldung:
**17.05.89 Patentblatt 89/20**

(45) Bekanntmachung des Hinweises auf die
Patenterteilung:
**23.10.91 Patentblatt 91/43**

(84) Benannte Vertragsstaaten:
**AT DE FR GB IT**

(56) Entgegenhaltungen:
**EP-A- 0 119 321**
**EP-A- 0 234 811**
**FR-A- 2 551 655**

(73) Patentinhaber: **GEBRÜDER SULZER AKTIENGE-
SELLSCHAFT**
**Zürcherstrasse 9**
**CH-8401 Winterthur(CH)**

(72) Erfinder: **Frey, Otto**
**Wallrütistrasse 56**
**CH-8400 Winterthur(CH)**
Erfinder: **Willi, Roland**
**Unterdorfstrasse 24**
**CH-8543 Stadel(CH)**

**Beschreibung**

Die Erfindung betrifft eine Hüftgelenkspfanne, bei der die Pfannenschale für die Aufnahme eines Gelenkkopfes in einen metallischen Schalenkörper eingelassen ist.

Wegen der guten Gleiteigenschaften verwendet man bei Totalprothesen für Hüftgelenke in letzter Zeit vermehrt wieder Gleitpaarungen, bei denen eine metallischer Gelenkkopf in eine ebenfalls aus einer polierten Metalloberfläche bestehenden Pfannenschale gelagert ist. Hüftgelenkspfannen mit metallischen Schalenkörpern, in die die Pfannenschale eingearbeitet ist, sind beispielsweise aus der DE-PS 34 46 048 bekannt.

Im "Normalbetrieb" einer solchen Totalprothese verteilen sich die Druckkräfte zwischen Gelenkkopf und Pfannenschale flächenhaft über "grössere" Bereiche der Oberfläche beider Elemente. Kommt es jedoch zu Luxationen, bei denen der Gelenkkopf aus der Pfannenschale "ausgekugelt" wird, so entstehen auf der Kugeloberfläche örtlich sehr begrenzte Linien- bzw. Punktbelastungen, die leicht zu Beschädigungen der polierten Kugeloberfläche führen können.

Aufgabe der Erfindung ist es, die Gefahr von Beschädigungen der Oberfläche des Gelenkkopfes bei Luxationen des Gelenkes zumindest zu verringern. Erfindungsgemäss wird diese Aufgabe dadurch gelöst, dass der äquatoriale Rand der Pfannenschale von einem Kunststoffring abgedeckt ist, dessen Innendurchmesser dem Innendurchmesser der Pfannenschale entspricht.

Das relativ zum metallischen Schalenkörper weiche Material des Kunststoffringes verhindert, dass die Kugeloberfläche durch örtliche Belastungsspitzen besonders bei punktförmigen Belastungen infolge einer Luxation des Gelenkes angegriffen und beschädigt wird.

Um auch Beschädigungen des Kunststoffringes möglichst in Grenzen zu halten und um ein automatisches Reponieren, d.h. ein "Zurückspringen" eines luxierten Gelenkkopfes in die Pfannenschale zu begünstigen, ist es zweckmässig, wenn der Innendurchmesser des Kunststoffringes sich nach aussen erweitert.

Bei einer besonders vorteilhaften Ausführungsform der neuen Hüftgelenkspfanne kann der metallische Schalenkörper in einen Kunststoff-Grundkörper eingesetzt sein, der auf seiner der Verankerung im Beckenknochen dienenden Aussenseite mit einer porösen Oberflächenstruktur in Form eines mehrlagigen Drahtnetzes versehen ist; der Kunststoff-Grundkörper kann jedoch auch in eine Metallschale eingebracht werden, wo er dann als stossdämpfendes Element wirkt.

Die Verbindung zwischen dem Kunststoffring und dem Schalenkörper wird konstruktiv besonders einfach, wenn der Kunststoffring in einer Ausnehmung des Grundkörpers gelagert und über einen Schappverschluss aussen am Schalenkörper gehalten ist; dabei kann zusätzlich der Schalenkörper ebenfalls durch einen Schappverschluss im Grundkörper gehalten sein.

Um ein Eindringen von Körperflüssigkeit in Spalten zwischen dem Kunststoffring und dem Schalenkörper auf einfache Weise zu verhindern, ist es schliesslich möglich, den Aussenmantel des Kunststoffringes und/oder die Gegenfläche dazu im Schalenkörper konisch auszubilden.

Im folgenden wird die Erfindung anhand eines Ausführungsbeispiels im Zusammenhang mit der Zeichnung näher erläutert.

Fig. 1     zeigt eine Ausführungsform der neuen Hüftgelenkspfanne in einem Meridianschnitt;

Fig. 2     ist ein Ausschnitt aus Fig. 1 in vergrössertem Massstab.

Die neue Hüftgelenkspfanne hat einen Grundkörper 1 aus Kunststoff, dessen halbkugelförmige Aussenfläche mit einem mehrlagigen porösen Drahtnetz 2 belegt ist; dieses dient als Verankerungsstruktur entweder für das Einwachsen von Knochengewebe oder für eine mechanische "Verklammerung" mit einem Knochenzementbett.

Die eigentliche Pfannenschale 3 für die Aufnahme des nicht gezeigten Gelenkkopfes einer Femurkopfprothese befindet sich in einem metallischen Schalenkörper 4, der im Grundkörper 1 über einen Schnappverschluss 6 gehalten ist.

Zum Aequator der Hüftgelenkspfanne hin ist dem Schalenkörper 4 ein Kunststoffring 7 vorgelagert. Dieser Ring, dessen Innendurchmesser sich zunächst vom Innendurchmesser der Pfannenschale weg in einer konvexen Wölbung und anschliessend geradlinig beim "Fortschreiten" aus der Pfannenschale 3 heraus nach aussen erweitert, bildet den "äusseren" Abschluss der Pfannenschale 3, auf dem sich der Gelenkkopf im Falle einer Gelenkluxation abstützt.

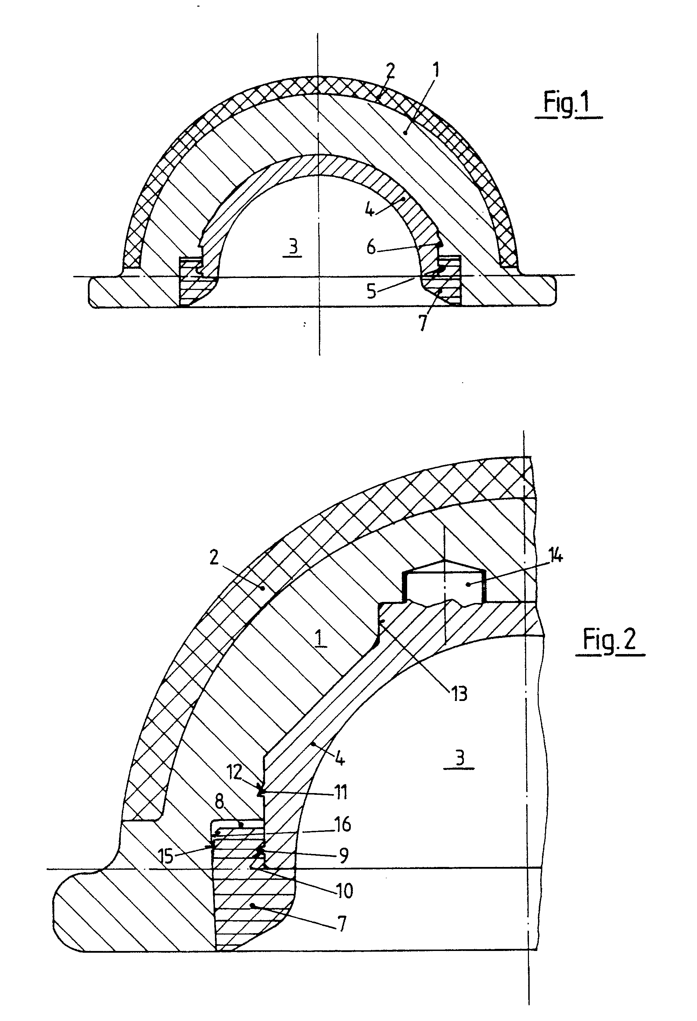

Der Kunststoffring 7, der in einer Ausnehmung 8 (Fig. 2) des Grundkörpers 1 gelagert ist, ist am Schalenkörper 4 aussen durch einen Schnappverschluss 5 gehalten. Bei der Montage von Schalenkörper 4 und Ring 7 wird dieser zunächst am Schalenkörper 4 befestigt; daraufhin werden beide gemeinsam in den Grundkörper 1 eingedrückt, bis der Schappverschluss 6 einrastet.

Während der Schnappverschluss 5 aus einem ringförmigen Vorsprung 9 (Fig. 2) an der Aussenseite des Schalenkörpers 4 und einer entsprechenden Nut 10 im Kunststoffring 7 besteht, greift bei dem Schnappverschluss 6 eine ringförmige Nase 11 in eine entsprechende Vertiefung 12 im Hohlraum des Grundkörpers 1 ein.

Ein Verkanten des Schalenkörpers 4 relativ zum Grundkörper 1 beim Einpressen wird durch eine zylindrische Führung 13 im Scheitelbereich des Schalenkörpers 4 bzw. des Grundkörpers 1 verhindert. Als Verdrehsicherung zwischen beiden Körpern 1 und 4 ist ebenfalls im Scheitelbereich aus dem Schalenkörper 4 vorstehender in den Grundkörper 1 eingreifender Zapfen 14 vorgesehen.

Um ein Eindringen von Körperflüssigkeit in die Ausnehmung 8 zu verhindern, verlaufen die Ringmantelfläche 15 am Kunststoffring 7 und/oder die Gegenfläche 16 der Ausnehmung 8 derart konisch, dass der äussere Rand dieser Flächen über eine gewisse Höhe gegeneinander gepresst werden.

Selbstverständlich ist die Erfindung nicht nur bei Konstruktionen anwendbar, bei denen der Schalenkörper 4 und der Grundkörper 1 getrennte Teile sind, sondern auch bei solchen, bei denen die Pfannenschale 3 direkt in einem Pfannenkörper angeordnet ist, der gegebenenfalls aussen noch von einem Verankerungsrring oder einer Aussenschale umgeben sein kann.

## Patentansprüche

1. Hüftgelenkspfanne, bei der die Pfannenschale für die Aufnahme eines Gelenkkopfes in einen metallischen Schalenkörper eingelassen ist, **dadurch gekennzeichnet,** dass der äquatoriale Rand der Pfannenschale (3) von einem Kunststoffring (7) abgedeckt ist, dessen Innendurchmesser dem Innendurchmesser der Pfannenschale (3) entspricht.

2. Hüftgelenkspfanne nach Anspruch 1, dadurch gekennzeichnet, dass der Innendurchmesser des Kunststoffringes (7) sich nach aussen erweitert.

3. Hüftgelenkspfanne nach Anspruch 1, dadurch gekennzeichnet, dass der metallische Schalenkörper (4) in einen Kunststoff-Grundkörper (1) eingesetzt ist, der auf seiner der Verankerung im Beckenknochen dienenden Aussenseite mit einer porösen Oberflächenstruktur in Form eines mehrlagigen Drahtnetzes (2) versehen ist.

4. Hüftgelenkspfanne nach Anspruch 3, dadurch gekennzeichnet, dass der Kunststoffring (7) in einer Ausnehmung (8) des Grundkörpers (1) gelagert und über einen Schnappverschluss (5) aussen am Schalenkörper (4) gehalten ist.

5. Hüftgelenkspfanne nach Anspruch 3, dadurch gekennzeichnet, dass der Schalenkörper (4) im Grundkörper (1) durch einen weiteren Schnappverschluss (6) gehalten ist.

6. Hüftgelenkspfanne nach Anspruch 1 oder 3, dadurch gekennzeichnet, dass der Aussenmantel (15) des Kunststoffringes (7) und/oder die zugehörige Gegenfläche (16) im Schalenkörper (4) konisch verlaufen.

## Claims

1. An acetabulum in which the shell receiving a femoral head is let into a metallic shell body, characterised in that the equatorial edge of the shell (3) is covered by a plastics ring (7) whose internal diameter corresponds to the internal diameter of the shell (3).

2. An acetabulum according to claim 1, characterised in that the inner diameter of the plastics ring (7) widens outwardly.

3. An acetabulum according to claim 1, characterised in that the metal shell body (4) is inserted into a plastics main or parent body (1) having a porous surface structure in the form of a multilayer wire mesh (2) on its outside, the same serving for fixing in the pelvis bone.

4. An acetabulum according to claim 3, characterised in that the plastics ring (7) is mounted in a recess (8) in the parent body (1) and is retained externally on the shell body (4) by a snap fastening (5).

5. An acetabulum according to claim 3, characterised in that the shell body (4) is retained in the parent body (1) by another snap fastening (16).

6. An acetabulum according to claim 1 or 3, characterised in that the outer envelope (15) of the plastics ring (7) and/or the associated companion surface (16) in the shell body (4) extend and/or extends conically.

## Revendications

1. Cavité cotyloïde dans laquelle la coque acétabulaire destinée à recevoir une tête d'articulation est encastrée dans un corps de coque métallique, caractérisé en ce que le bord équatorial de la coque acétabulaire (3) est recouvert par un anneau en matière plastique (7) dont le diamètre intérieur correspond au diamètre intérieur de la coque acétabulaire (3).

2. Cavité cotyloïde selon la revendication 1, caractérisée en ce que le diamètre intérieur de l'anneau en matière plastique (7) s'élargit vers l'extérieur.

3. Cavité cotyloïde selon la revendication 1, caractérisée en ce que le corps de coque (4) métallique est inséré dans un corps de base (1) en matière plastique qui est pourvu d'une structure superficielle poreuse en forme de grillage métallique (2) à plusieurs couches, sur le côté extérieur servant à l'ancrage dans l'os iliaque.

4. Cavité cotyloïde selon la revendication 3, caractérisée en ce que l'anneau en matière plastique (7) est monté dans un évidement (8) du corps de base (1) et est maintenu extérieurement contre le corps de coque (4), par une fermeture clipsée (5).

5. Cavité cotyloïde selon la revendication 3, caractérisée en ce que le corps de coque (4) est maintenu dans le corps de base (1) par une autre fermeture clipsée (6).

6. Cavité cotyloïde selon la revendication 1 ou 3, caractérisée en ce que l'enveloppe extérieure (15) de l'anneau en matière plastique (7) et/ou la contre-surface correspondante (16) s'étendent coniquement dans le corps de coque (4).

Fig.1

Fig.2